# FASCICULE DE BREVET EUROPEEN

(11) **EP 0 925 806 B1**
(45) Date de publication et mention de la délivrance du brevet: **26.05.2004**
(21) Numéro de dépôt: 98403271.4
(22) Date de dépôt: 23.12.1998
(51) Int. Cl.: A61N 1/368, A61N 1/39

(54) **Dispositif médical implantable actif, notamment stimulateur cardiaque, défibrillateur et/ou cardioverteur, de type multisite configurable**
Implantierbare aktive medizinische Einrichtung, insbesondere Herzstimulator, Defibrillator und/oder Kardiovertierer, mit einer konfigurierbaren Elektrodenverteilung
Configurable multisite implantable active medical device, especially heart stimulator, defibrillator and/or cardioverter

(30) Priorité: 23.12.1997 FR 9716378
(43) Date de publication de la demande: 30.06.1999
(73) Titulaire: ELA MEDICAL (Société anonyme), 92541 Montrouge (FR)
(72) Inventeur: Legay, Thierry, 91640 Fontenay les Briis (FR); Bouhour, Anne, 92410 Ville d'Avray (FR); Ripart, Alain, 91190 Gif sur Yvette (FR)
(74) Mandataire: Dupuis-Latour, Dominique

(56) Documents cités:
- EP-A- 0 308 536
- EP-A- 0 598 617
- EP-A- 0 813 889
- GB-A- 2 079 610
- US-A- 5 360 435

## Description

L'invention concerne les "dispositifs médicaux implantables actifs" tels que définis par la directive 90/385/CEE du 20 juin 1990 du Conseil des communautés européennes, plus précisément les dispositifs stimulateurs cardiaques, défibrillateurs et/ou cardioverteurs permettant de délivrer au coeur des impulsions électriques de faible énergie pour le traitement des troubles du rythme cardiaque.

Elle concerne plus particulièrement les prothèses dites "multisite", c'est-à-dire les prothèses dans lesquelles des électrodes sont placées en une pluralité de sites respectifs distincts, avec au moins deux sites ventriculaires. Il peut s'agir d'une prothèse de type "simple chambre" (double stimulation ventriculaire), triple chambre (stimulation atriale droite et double stimulation ventriculaire) ou même quadruple chambre (double stimulation atriale et double stimulation ventriculaire).

Ces électrodes sont reliées à des bornes indépendantes du stimulateur de manière à permettre la détection de potentiels de dépolarisation ainsi que l'application d'impulsions de stimulation.

Le EP-A-0 030 897 décrit un tel stimulateur multisite, dans lequel les deux bornes des électrodes de stimulation sont implantées en deux sites prédéterminés appartenant à une même cavité cardiaque, notamment le ventricule. Un dispositif de commutation permet de choisir le mode de fonctionnement du stimulateur parmi divers modes (bipolaire, monopolaire double, double monopolaire ou bifocal ventriculaire), sans toutefois modifier l'implantation des électrodes ni le choix des sites de stimulation.

Le EP-A-0 598 617 décrit un défibrillateur-cardioverteur implantable pourvu d'un circuit de commutation permettant de sélectionner de manière programmable la polarité des impulsions de choc appliquées aux diverses électrodes de defibrillation lors du déclenchement d'une thérapie antitachycardique.

Le document EP-A-0 813 889, est un document selon l'Art 54(3) qui décrit un dispositif médical implantable actif. Seule la caractéristiques "les moyens d'évaluation pour la recherche de la configuration de stimulation optimale" n'est pas décrite dans le document EP-A-0 813 889.

L'un des buts de l'invention est de proposer un stimulateur cardiaque (ou un circuit de stimulation d'un défibrillateur ou cardioverteur) de type multisite permettant, sans accroissement notable de la complexité de la circuiterie du stimulateur, de modifier le choix des sites en recherchant une configuration optimale de stimulation.

En effet, lorsque l'on modifie après un certain temps la configuration des sites de stimulation, on constate une meilleure récupération du tonus musculaire du myocarde, et donc une réduction de l'effort à fournir par celui-ci. Le but est d'avoir plusieurs pôles de stimulation pour les cardiopathies dilatées, et le fait de pouvoir modifier la configuration est un avantage pratique.

Toutefois, la multiplication du nombre de sites entraînerait une multiplication corrélative du nombre d'étages de stimulation et de chaînes de détection, qui se heurterait rapidement à des contraintes de volume disponible à l'intérieur du boîtier du stimulateur à raison de l'augmentation du nombre des composants. En outre et surtout, l'augmentation du nombre de composants induirait un accroissement important de la consommation du stimulateur, susceptible de réduire la durée de vie nominale de celui-ci en-deçà des limites exigées.

L'invention se propose de résoudre ces difficultés, en proposant un stimulateur doté de capacités de stimulation/détection multisite susceptible de stimuler et détecter selon des configurations multiples et modifiables à volonté tout en minimisant la quantité de composants nécessaire, ceci afin de réaliser un compromis entre, d'une part, les exigences cliniques et, d'autre part, les contraintes de volume à l'intérieur du boîtier et les contraintes de consommation des circuits.

À cet effet, le dispositif médical implantable actif de l'invention, dans lequel des électrodes sont placées en au moins trois sites myocardiques distincts de stimulation, dont au moins un site ventriculaire de stimulation/détection, ces électrodes étant reliées à des bornes indépendantes du dispositif de manière à permettre l'application d'impulsions de stimulation produites par une pluralité d'étages de stimulation distincts, en nombre au plus égal au nombre desdits sites de stimulation, est caractérisé en ce qu'il comprend des moyens de commutation pour relier sélectivement et selon diverses configurations possibles de stimulation les étages de stimulation, ou certains de ceux-ci, aux divers sites de stimulation, ou à certains de ceux-ci, et des moyens de commande des moyens de commutation, pour modifier la configuration de stimulation de manière à rechercher celle considérée comme optimale, et en ce qu'il est en outre prévu des moyens d'évaluation pour la recherche de ladite configuration optimale.

Ce dispositif peut également comprendre au moins deux étages distincts de détection d'activité électrique spontanée dans une cavité cardiaque, les moyens de commutation comprenant en outre des moyens aptes à relier sélectivement et selon diverses configurations possibles de détection ces étages de détection, ou certains de ceux-ci, aux divers sites de stimulation, ou à certains de ceux-ci, en propre ou concurremment avec une liaison à un étage de stimulation.

Avantageusement, les moyens de commande des moyens de commutation peuvent opérer dynamiquement en cours de fonctionnement du dispositif de manière à modifier les configurations de stimulation et/ou de détection.

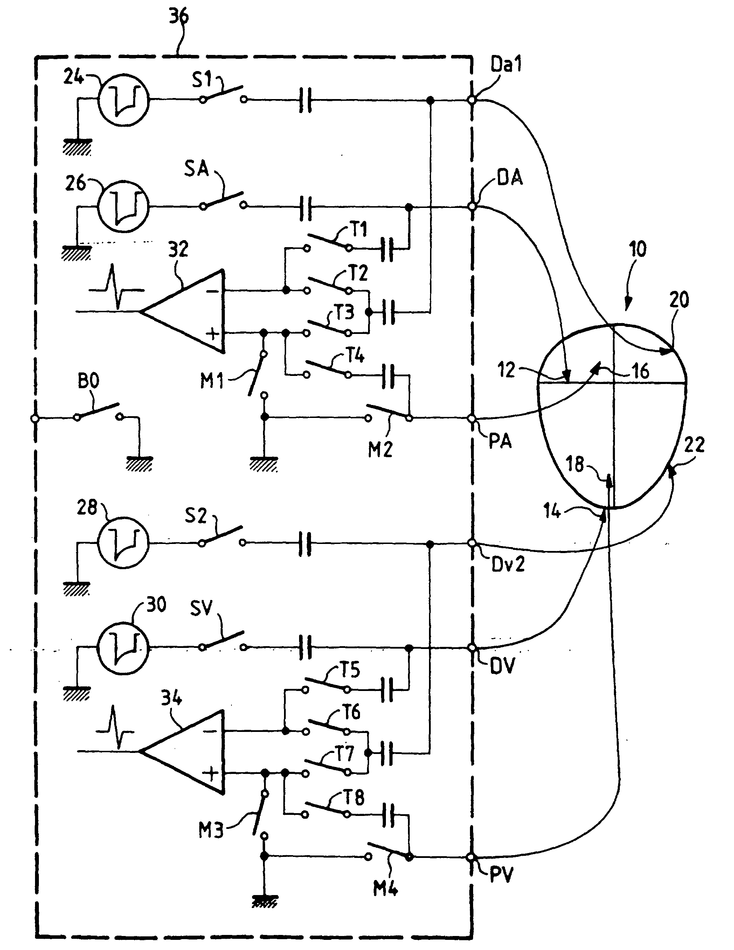

D'autres caractéristiques et avantages de l'invention apparaîtront à la lecture de la description détaillée ci-dessous d'un exemple de réalisation, en référence à la figure unique annexée qui est un schéma synoptique d'un stimulateur réalisé selon les enseignements de l'invention, associé à une pluralité de sondes implantées en divers sites d'un myocarde.

Sur la figure, la référence 10 désigne, de façon générale et schématique, le myocarde sur lequel sont implantées diverses sondes en une pluralité de sites 12, 14, 16, 18, 20 et 22.

Il peut s'agir d'électrodes en contact effectif avec le myocarde, telles que les électrodes 12, 14, 20 et 22 qui sont en contact avec chacune des quatre cavités du myocarde ; il peut s'agir également d'électrodes flottantes, telles que les électrodes 16 et 18, qui servent alors de référence de potentiel pour détecter des signaux ou stimuler, lorsque cette stimulation est opérée en mode bipolaire. Ces électrodes flottantes peuvent par exemple être constituées par l'électrode proximale d'une sonde bipolaire, dont l'extrémité distale est, quant à elle, en contact avec la cavité.

Ainsi, dans la configuration d'électrodes illustrée sur la figure, les électrodes 12 et 16 sont celles d'une sonde bipolaire implantée dans l'oreillette droite, les électrodes 14 et 18 sont celles d'une sonde bipolaire implantée dans le ventricule droit, l'électrode 20 est celle d'une sonde monopolaire implantée dans l'oreillette gauche et l'électrode 22 est celle d'une sonde monopolaire implantée dans le ventricule gauche.

Cette configuration n'est cependant en aucune façon limitative en ce qui concerne le nombre d'électrodes aussi bien que leur configuration, dès lors que l'on dispose d'au moins trois sites de stimulation/détection, dont au moins un dans le ventricule.

Par ailleurs, les sondes ne sont pas nécessairement placées dans les cavités cardiaques (électrodes en contact avec le myocarde ou électrodes flottantes), mais peuvent également être situées dans des lieux permettant de stimuler de manière indirecte les cavités, par exemple implantées dans un sinus coronaire, ou encore à l'extérieur du myocarde (sonde épicardique).

L'une des caractéristiques de l'invention est en effet de pouvoir disposer d'un système où les électrodes ne sont plus dédiées à des cavités cardiaques particulières, mais peuvent être placées dans n'importe quelle cavité cardiaque ; on peut même placer toutes les électrodes dans la même cavité, par exemple dans le ventricule droit avec une électrode dans le septum pour pouvoir stimuler le ventricule gauche et/ou à la croisée des cavités, etc.

En ce qui concerne le stimulateur, dans l'exemple illustré celui-ci comporte (ici encore, de façon non limitative) quatre pôles indépendants permettant de délivrer une stimulation électrique paramétrable en amplitude et en largeur en quatre points du coeur et deux pôles, typiquement des électrodes proximales, servant de références de potentiel pour la stimulation et la détection.

Par convention, les six pôles du dispositif sont désignés en fonction de l'utilisation typique qui est celle illustrée, bien que ceci ne constitue en aucun cas une limite aux possibilités du système puisque chacun des pôles peut être relié à un site situé dans n'importe laquelle des cavités cardiaques. On désignera par :
― DA et DV (Distales Auriculaire et Ventriculaire) les pôles reliés aux électrodes 12 et 14,
― PA et PV (Proximales Auriculaire et Ventriculaire) les pôles reliés aux électrodes 16 et 18,
― Da1 (Distale auriculaire) le pôle relié à l'électrode 20, qui est le pôle additionnel n° 1 par rapport à un dispositif double chambre, et
― Dv2 (Distale ventriculaire) le pôle relié à l'électrode 22, qui est le pôle additionnel n° 2 par rapport à un dispositif double chambre.

Le stimulateur comporte quatre étages de stimulation 24, 26, 28 et 30 et deux chaînes de détection, avec des amplificateurs respectifs 32 et 34.

Divers commutateurs électroniques SA, SV, S1, S2, M1-M4 et T1-T8 sont connectés de la manière représentée sur la figure entre les étages de stimulation 24, 26, 28 et 30 et les étages de détection 32 et 34, d'une part, et les six pôles DA, PA, Da1, DV, PV, Dv2, d'autre part. Ils permettent d'aiguiller chacun de ces six pôles vers un étage 24 à 34 afin de réaliser diverses configurations de stimulation et diverses configurations de détection du signal cardiaque.

Il est également prévu un commutateur B0 permettant de connecter le boîtier métallique 36 du dispositif à la masse du système, lorsque l'on souhaite réaliser une stimulation ou une détection entre un pôle endocavitaire et la masse du boîtier.

On va maintenant indiquer les diverses configurations de stimulation et de détection qu'il est possible de réaliser au moyen de ces différents commutateurs.

Les divers modes de détection et de stimulation seront désignés de la manière suivante :
― "détection unipolaire" : détection entre un pôle endocavitaire et le boîtier,
― "détection bipolaire" : détection différentielle entre deux pôles endocavitaires (le boîtier constituant une référence pour le mode commun),
― "détection tripolaire" : détection sur deux pôles endocavitaires reliés entre eux, par rapport à un troisième pôle endocavitaire (le boîtier constituant une référence pour le mode commun),
― "stimulation unipolaire" : stimulation entre un pôle endocavitaire et le boîtier,
― "stimulation bipolaire" : stimulation entre deux pôles endocavitaires dont un est la masse (boîtier en l'air).

De plus, comme dans le cas d'un stimulateur classique DDD (double chambre), le terme "bipolaire" fait référence à deux pôles endocavitaires situés dans la même cavité mais qu'avec un stimulateur multisite (MS), ce n'est plus le cas, on appellera par convention "quasi-bipolaire" une configuration faisant intervenir deux électrodes endocavitaires dans deux cavités différentes, et de même pour "quasi-tripolaire".

Les différentes possibilités de configuration sont données par le tableau suivant, qui indique :
― la configuration considérée
― les pôles (électrodes) impliqués,
― le fait qu'il s'agisse d'une configuration déjà connue en tant que telle dans un stimulateur double chambre classique (DDD), ou d'une configuration nouvelle, propre au dispositif multisite de la présente invention (MS),
― les commutateurs devant être fermés,
― les commutateurs devant être ouverts.

| Configuration | | Type | Fermés | Ouverts |
|---|---|---|---|---|
| Dét. unip. | Da1 | MS | B0, M1, T2 | M2, T1, T3, T4 |
| Dét. quasi-bip. | Da1/PA | MS | B0, T2, T4 | M1, M2, T1, T3 |
| Dét. quasi-bip. | DA/Da1 | MS | B0, T1, T3 | M1, M2, T2, T4 |
| Dét. quasi-trip. | [DA+Da1]/PA | MS | B0, T1, T2, T4 | M1, M2, T3 |
| Dét. bip. | DA/PA | DDD | B0, T1, T4 | M1, M2, T2, T3 |
| Dét. unip. | DA | DDD | B0, M1, T1 | M2, T2, T3, T4 |
| | | | | |
| Stim. unip. | Da1 | MS | B0, S1 | M2, M4 |
| Stim. quasi-bip. | Da1/PA | MS | M2, S1 | B0, M4 |
| Stim. unip. | DA | DDD | B0, SA | M2, M4 |
| Stim. bip. | DA/PA | DDD | M2, SA | B0, M4 |
| | | | | |
| Dét. unip. | Dv2 | MS | B0, M3, T6 | M4, T5, T7, T8 |
| Dét. quasi-bip. | Dv2/Pv | MS | B0, T6, T8 | M3, M4, T5, T7 |
| Dét. quasi-bip. | DV/Dv2 | MS | B0, T5, T7 | M3, M4, T6, T8 |
| Dét. quasi-trip. | [DV+Dv2]/PV | MS | B0, T5, T6, T8 | M3, M4, T7 |
| Dét. bip. | DV/PV | DDD | B0, T5, T8 | M3, M4, T6, T7 |
| Dét. unip. | DV | DDD | B0, M3, T5 | M4, T6, T7, T8 |
| | | | | |
| Stim. unip. | Dv2 | MS | B0, S2 | M2, M4 |
| Stim. quasi-bip. | Dv2/PV | MS | M4, S2 | B0, M2 |
| Stim. unip. | DV | DDD | B0, SV | M2, M4 |
| Stim. bip. | DV/PV | DDD | M4, SV | B0, M2 |

On voit ainsi que l'on dispose ― mais de façon non exhaustive ―, pour la stimulation, de huit configurations différentes dont quatre sont nouvelles et, pour la détection, de douze configurations possibles dont huit sont nouvelles.

En pratique, la localisation des électrodes dans toute partie des quatre cavités cardiaques est laissée au choix du praticien. La configuration de stimulation et celle de détection sont ensuite réalisées, soit conformément aux indications du praticien, soit de façon automatique par recherche d'une configuration optimale.

Une configuration est considérée comme meilleure si elle procure une amélioration des paramètres cardiaques tels que le débit. La recherche de cette configuration peut être déclenchée de façon manuelle (envoi d'ordres de commutation au dispositif par un programmateur externe sous contrôle d'un praticien) ou automatique (par exemple par une commande interne périodique du logiciel de pilotage du stimulateur), ce qui permettra d'explorer les diverses configurations possibles jusqu'à trouver la meilleure, que l'on qualifiera alors d'"optimale". Le paramètre cardiaque à optimiser peut être évalué par le praticien (par exemple par une technique Doppler externe), ou en interne par le stimulateur à partir des signaux cardiaques recueillis, par exemple par une technique telle que celle exposée dans le EP-A-0 862 927 (ELA Médical).

Les commutateurs peuvent par ailleurs être programmés de manière à modifier dynamiquement la configuration en cours de fonctionnement du stimulateur, afin d'adapter dynamiquement celle-ci pour toujours se placer dans la configuration la meilleure.

Les stimulations sur chacun des quatre pôles choisis sont contrôlés par un certain nombre de paramètres, notamment de délais :
― délai entre étages, à savoir entre l'étage auriculaire A et l'étage auriculaire V, par le délai atrio-ventriculaire (DAV) classique d'un stimulateur DDD,
― délai entre cavités d'un même étage, entre DA et Da1 ou entre DV et Dv2, les délais DA-Da1 et DV-Dv2 pouvant, suivant la programmation du stimulateur, être des délais Da1-DA ou Dv2-DV,
― suivant la localisation des sondes dans le coeur, que le praticien devra spécifier à l'implantation du dispositif, un certain nombre de nouveaux modes préférentiels de stimulation vont être proposés, accompagnés des paramètres programmables de couplage temporel. Les délais entre étages peuvent être par exemple programmables de 0 à 300 ms par pas de 8 ms, et de même pour les délais entre cavités ;
― pour chaque pôle capable de délivrer une stimulation électrique, des paramètres et d'amplitude d'impulsion sont accessibles de façon indépendante.

Un autre avantage du dispositif de l'invention est la diminution du nombre de sorties électriques sur le boîtier du stimulateur. Ainsi, dans un stimulateur "4 chambres" classique en configuration bipolaire pour chaque sonde, huit sorties sont nécessaires ; dans le cas de l'invention, six sorties seulement sont nécessaires, réduisant d'autant le nombre de traversées et la taille de la tête de connecteur du stimulateur. Dans le cas d'un système multisite ventriculaire double avec un étage auriculaire (configuration la plus courante), cinq sorties seulement seront nécessaires, à savoir DA, PA pour l'oreillette et PV, DV, Dv2 pour les ventricules.

## Revendications

1. Un dispositif médical implantable actif, notamment un stimulateur cardiaque défibrillateur et/ou cardioverteur, dans lequel des électrodes sont aptes à être placées en au moins trois sites myocardiques distincts de stimulation (12, 14, 20, 22), dont au moins un site ventriculaire de stimulation/détection (14, 22), ces électrodes étant reliées à des bornes indépendantes (DA, DV, Da1, Dv2) du dispositif de manière à permettre l'application d'impulsions de stimulation produites par une pluralité d'étages de stimulation distincts (24, 26, 28, 30), en nombre au plus égal au nombre desdits sites de stimulation,
le dispositif comprend des moyens de commutation (SA, SV, S1, S2) pour relier sélectivement et selon diverses configurations possibles de stimulation les étages de stimulation, ou certains de ceux-ci, aux divers electrodes aptes à être placées à lesdites sites de stimulation, ou à certains de ceux-ci, et des moyens de commande des moyens de commutation, pour modifier la configuration de stimulation de manière à rechercher celle considérée comme optimale, et en ce qu'il est en outre prévu des moyens d'évaluation pour la recherche de ladite configuration optimale.

2. Le dispositif médical de la revendication 1, comprenant en outre au moins deux étages distincts de détection (32, 34) d'activité électrique spontanée dans une cavité cardiaque, et dans lequel les moyens de commutation comprennent en outre des moyens (M1-M4, T1-T8) aptes à relier sélectivement et selon diverses configurations possibles de détection ces étages de détection, ou certains de ceux-ci, aux divers sites de stimulation, ou à certains de ceux-ci, en propre ou concurremment avec une liaison à un étage de stimulation.

3. Le dispositif de la revendication 1 ou 2, dans lequel les moyens de commande des moyens de commutation opèrent dynamiquement en cours de fonctionnement du dispositif de manière à modifier les configurations de stimulation et/ou de détection.

## Patentansprüche

1. Aktive implantierbare medizinische Vorrichtung, insbesondere ein Herzschrittmacher, Defibrillator und/oder Kardioverter, in welchem die Elektroden geeignet sind, in mindestens drei unterschiedlichen Stimulationsstellen (12, 14, 20, 22) platziert zu werden, von denen mindestens eine ventrikuläre Stelle zur Stimulation/Erfassung (14, 22) ist, wobei die Elektroden mit unabhängigen Anschlüssen (DA, DV, Da1, Dv2) der Vorrichtung verbunden sind, um die Anwendung von Stimulationsimpulsen zu ermöglichen, welche durch eine Vielzahl von unterschiedlichen Stimulationsstufen (24, 26, 28, 30) erzeugt werden, in der Anzahl höchstens gleich der Anzahl der Stimulationsstellen,
die Vorrichtung enthält Kommutationsmittel (SA, SV, S1, S2), um selektiv und entlang verschiedener möglicher Stimulationskonfigurationen die Stimulationsstufen zu verbinden, oder einige derselben, mit verschiedenen Elektroden, die geeignet sind, an den Stimulationsstellen platziert zu werden, oder mit einigen derselben, zu verbinden und Mittel zur Steuerung der Kommutationsmittel, um die Stimulationskonfiguration zu modifizieren, um die als optimal angesehene zu suchen, und dadurch, das femer Evaluierungsmittel für die Suche der optimalen Konfiguration vorgesehen sind.

2. Medizinische Vorrichtung nach Anspruch 1, umfassend ferner mindestens zwei unterschiedliche Erfassungsstufen (32, 34) der spontanen elektrischen Aktivität in einer Herzkammer, und in welcher die Kommutationsmittel ferner Mittel (M1-M4, T1-T8) umfassend, die geeignet sind, diese Erfassungsstufen selektiv und entlang verschiedener möglicher Erfassungskonfigurationen zu verbinden, oder einige derselben, mit verschiedenen Stimulationsstellen, oder mit einigen derselben, selbst oder gemeinsam mit einer Verbindung zu einer Stimulationsstufe, zu verbinden.

3. Vorrichtung gemäß Anspruch 1 oder 2, in welcher die Mittel zur Steuerung der Kommutationsmittel dynamisch während des Betriebs der Vorrichtung arbeiten, um die Konfigurationen zur Stimulation und/oder zur Erfassung zu modifizieren.

## Claims

1. An active implantable medical device, in particular a cardiac pacemaker, defibrillator and/or cardioverter, in which electrodes are adapted to be placed in at least three distinct myocardial stimulation sites (12, 14, 20, 22), of which at least one site is a ventricular stimulation/detection site (14, 22), said electrodes being connected to independent terminals (DA, DV, Da1, Dv2) of the device, in a manner as to allow the application of stimulation pulses produced by a plurality of distinct stimulation stages (24, 26, 28, 30), the number of which is at most equal to the number of said stimulation sites,
the device comprising switching means (SA, SV, S1, S2) for connecting selectively and according to a plurality of possible stimulation arrangements the stimulation stages, or certain ones of them, to the various electrodes adapted to be placed in said stimulation sites, or to certain ones of them, and means for controlling the switching means, for modifying the stimulation arrangement in a manner as to search the one to be considered as the optimal configuration, and in that means are provided for evaluating the search of said optimal arrangement.

2. The medical device of claim 1, further comprising at least two distinct stages (32, 34) for the detection of spontaneous electrical activity in a cardiac cavity, and in which the switching means further comprise means (M1-M4, T1-T8) for connecting selectively and according to various possible detection arrangements said detection stages, or certain ones of them, to the various stimulation sites, or to certain ones of them, exclusively or concurrently with a connection to a stimulation stage.

3. The device of claim 1 in which the control means of the commutation means operate dynamically during functioning of the device in order to modify the of stimulation and/or detection arrangements.
